# FASCICULE DE BREVET EUROPEEN

(11) **EP 3 826 510 B1**
(45) Date de publication et mention de la délivrance du brevet: **07.09.2022**
(21) Numéro de dépôt: 19740410.6
(22) Date de dépôt: 22.07.2019
(51) Int. Cl.: A47C 27/10, A61B 17/02, A61F 2/34, A61B 17/00, A61F 2/02, E04H 15/20

(54) **STRUCTURE PNEUMATIQUE ET PROCÉDÉ DE FABRICATION ASSOCIÉ**
DRUCKLUFTSTRUKTUR UND ZUGEHÖRIGES HERSTELLUNGSVERFAHREN
PNEUMATIC STRUCTURE AND ASSOCIATED PRODUCTION METHOD

(30) Priorité: 23.07.2018 FR 1856800
(43) Date de publication de la demande: 02.06.2021
(73) Titulaire: Paris Sciences et Lettres, 75006 Paris (FR); Centre national de la recherche scientifique, 75016 Paris (FR); Ecole Supérieure de Physique et de Chimie Industrielles de la Ville de Paris, 75005 Paris (FR); Sorbonne Université, 75006 Paris (FR); Université Paris Cité, 75006 Paris (FR)
(72) Inventeur: SIEFERT, Emmanuel, 75018 PARIS (FR); ROMAN, Benoît, 94200 IVRY SUR SEINE (FR); BICO, José, 75012 PARIS (FR); REYSSAT, Etienne, 92160 ANTONY (FR)
(74) Mandataire: Lavoix
(86) Numéro de dépôt international: PCT/EP2019/069707
(87) Numéro de publication internationale: WO 2020/020839

(56) Documents cités:
- WO-A2-03/094666
- CN-A- 102 578 716
- FR-A1- 2 968 726
- GB-A- 1 268 368
- US-A1- 2010 143 130
- US-A1- 2017 035 498

## Description

La présente invention concerne une structure pneumatique comportant un corps déformable élastiquement définissant au moins un réseau de cavités internes, chaque cavité interne présentant un contour fermé dans au moins une section de la cavité interne,
chaque cavité interne étant propre à être mise en pression pour faire passer le corps déformable élastiquement d'une configuration de repos à au moins une configuration mise en pression.

Une telle structure est destinée à être utilisée pour former des objets tridimensionnels de structure prédéfinie, à partir d'une forme de référence, par application d'une pression interne positive ou négative dans des cavités internes du corps déformable élastiquement.

Les objets tridimensionnels sont par exemple des équipements biomédicaux, des équipements de loisir ou de rééducation, des parties de meubles, ou encore des structures industrielles.

Dans de nombreux domaines, il est souhaitable de disposer de structures présentant au repos une configuration contractée compacte, et occupant lors de leur utilisation, une configuration déployée, dans laquelle la structure présente un volume d'usage.

Dans certains cas, la structure est pneumatique. Le passage de la configuration de repos à la configuration déployée est réalisé en gonflant la structure avec un fluide.

Les structures pneumatiques présentent des propriétés mécaniques très limitées, notamment en raison du grand volume occupé par les cavités internes du corps par rapport à la matière présente dans les parois du corps.

En outre, ces structures tridimensionnelles sont figées et posent généralement des difficultés de pliage.

D'autres structures pneumatiques sont décrites par exemple dans US 9,464,642, WO 03/094666, US 2010/143130, CN 102578716 et GB 1 268 368.

Ces structures présentent généralement une pluralité de canaux internes, qui, après gonflage, engendrent des déformations substantielles de la structure, pour qu'elle se courbe, qu'elle s'allonge, qu'elle se contracte, ou qu'elle se torde lors d'une mise en pression.

Les structures décrites dans US 9,464,642 sont par exemple utilisées comme actionneurs pneumatiques. Toutefois, lors du gonflage de la structure, la déformation des cavités interne est très importante. Elle se produit généralement dans une seule direction et elle induit une expansion au niveau des cavités qui déforme fortement la structure, bien au-delà du rayon de courbure macroscopique occupé par la structure au niveau de la cavité.

Ainsi les formes obtenues par le gonflement de la structure sont essentiellement limitées par une cinématique de type flexion-extension, puisque la déformation est quasi unidimensionnelle, et l'aspect extérieur de la structure est fondamentalement modifié par la présence de bulles gonflées et fortement déformées au niveau des cavités.

Un but de l'invention est d'obtenir une structure pneumatique très simplement activable pour changer rapidement de forme et obtenir une forme contrôlée et reproductible qui s'adapte à l'application souhaitée.

A cet effet, l'invention a pour objet une structure selon la revendication 1.

La structure selon l'invention peut comprendre l'une ou plusieurs des caractéristiques des revendications 2 à 13, prises isolément ou suivant toute combinaison techniquement possible.

L'invention a également pour objet un procédé de fabrication d'une structure telle que définie plus haut selon la revendication 14.

Le procédé selon l'invention peut être selon la revendication 15.

L'invention sera mieux comprise à la lecture de la description qui va suivre, donnée uniquement à titre d'exemple, et faite en se référant aux dessins annexés, sur lesquels :
- la figure 1 est une vue d'une première structure pneumatique selon l'invention, dans une configuration de repos ;
- la figure 2 est une vue analogue à la figure 1, dans une première configuration mise en pression, dans laquelle une pression positive est appliquée dans la structure pneumatique ;
- la figure 3 est une vue analogue à la figure 1, dans une deuxième configuration mise en pression, dans laquelle une pression négative est appliquée dans la structure pneumatique ;
- la figure 4 est une vue, prise en coupe dans un plan médian de la structure de la figure 1, illustrant la configuration des cavités internes présentes dans la structure ;
- la figure 5 est une vue schématique en coupe d'un détail de la structure perpendiculairement au plan médian, dans la configuration de repos ;
- la figure 6 est une vue analogue à la figure 5, dans la première configuration mise en pression ;
- les figures 7 à 15 illustrent des variantes de structures selon l'invention dans la configuration de repos et dans une configuration mise en pression ;
- les figures 16 à 18 illustrent un premier exemple de structure selon l'invention munie d'une armature interne rigidifiante ;
- les figures 19 à 20 illustrent un deuxième exemple d'armature interne rigidifiante ;
- les figures 21 à 23 illustrent un troisième exemple d'armature interne rigidifiante
- la figure 24 illustre une autre structure pneumatique selon l'invention ;
- la figure 25 illustre encore une autre structure pneumatique selon l'invention ;
- les figures 26 et 27 illustrent un quatrième exemple d'armature interne rigidifiante;
- les figures 28 et 29 illustrent un cinquième exemple d'armature interne rigidifiante.

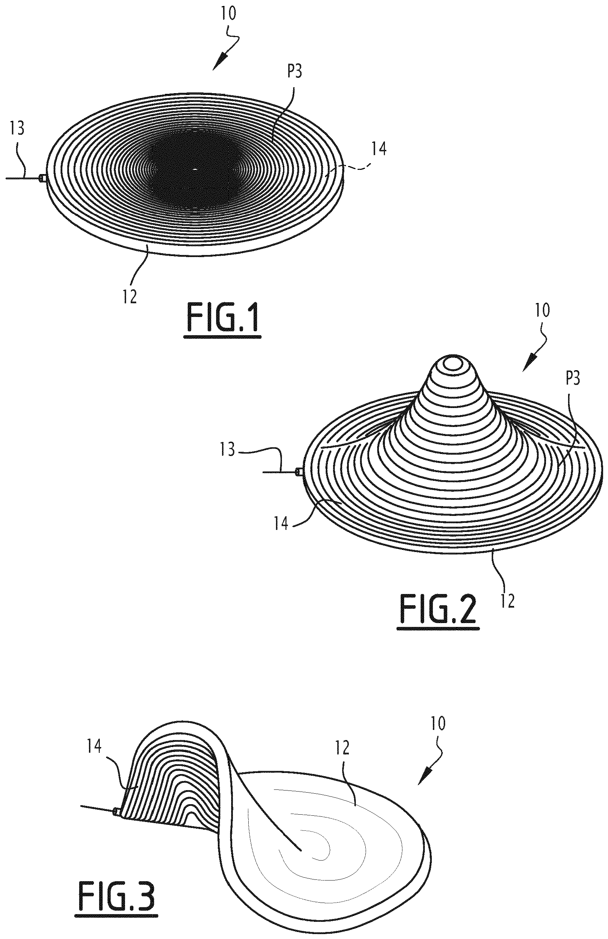

Une première structure pneumatique 10 selon l'invention est illustrée schématiquement sur les figures 1 à 6.

La structure 10 comporte un corps déformable élastiquement 12 définissant intérieurement une pluralité de cavités internes 14, propres à être mises en pression pour faire passer le corps déformable élastiquement 12 d'une configuration de repos, représentée sur la figure 1, à une pluralité de configurations mises en pression, dont deux exemples sont représentés sur les figures 2 à 3.

La structure 10 comporte en outre au moins un port 13 de mise en pression, communiquant avec les cavités internes 14 pour permettre la mise en pression des cavités 14.

Comme on va le décrire en détail plus bas, le corps déformable élastiquement 12 est configuré, par la structure des cavités internes 14 qu'il contient, pour se déformer de manière réversible entre sa configuration de repos, avantageusement plane, et chaque configuration mise en pression, dans laquelle il adopte une forme tridimensionnelle présentant au moins en partie une courbure gaussienne non nulle, la métrique macroscopique du corps déformable élastiquement 12 dans la configuration mise en pression étant différente de la métrique macroscopique du corps déformable élastiquement 12 dans la configuration de repos.

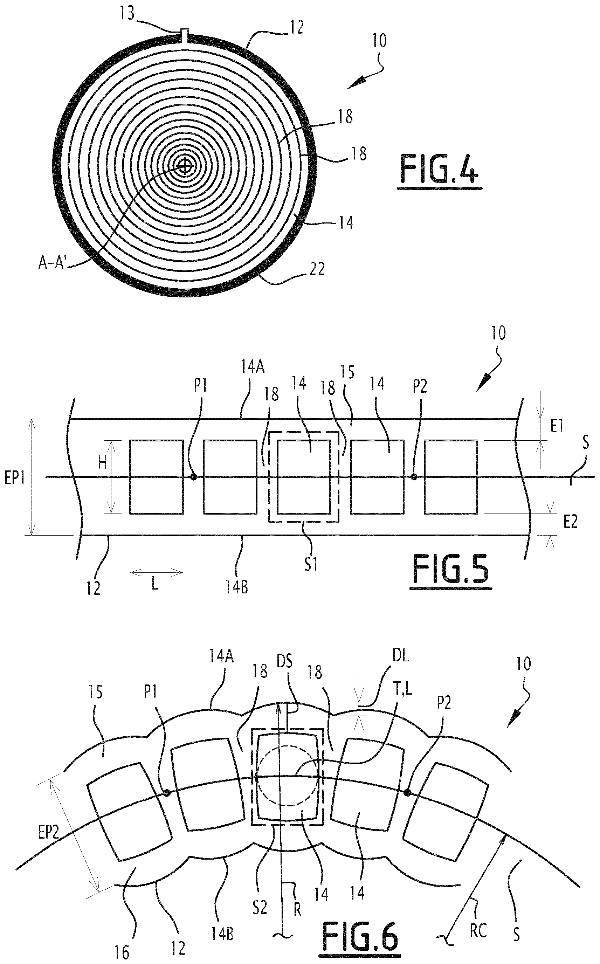

Par « métrique macroscopique du corps déformable élastiquement », on entend l'ensemble des distances séparant deux points P1, P2 différents de la surface médiane S (voir figures 5 et 6) définie au sein du corps déformable élastiquement 12 entre sa surface supérieure 14A et sa surface inférieure 14B.

Ainsi, au moins certaines distances entre des points différents P1, P2 de la surface médiane S ont varié lors du passage de la configuration de repos à la configuration mise en pression.

Plus généralement, la métrique macroscopique est avantageusement définie comme l'ensemble des distances entre les centres des cavités internes 14.

Dans le cas d'une cavité non-convexe, celle-ci est décomposée en plusieurs souscavités dont les centres sont autant de points de référence pour cette définition.

Ainsi, si la métrique macroscopique est modifiée, cela signifie qu'il existe un découpage pour lequel la distance entre les centres de deux cavités au moins est modifiée.

De même, en référence aux figures 1 et 2, en au moins un point de la surface médiane S, la courbure de Gauss est nulle dans la configuration de repos et est non nulle, par exemple positive ou négative, dans la configuration mise en pression.

Par « courbure de Gauss » ou « courbure gaussienne », on entend le produit des rayons de courbure principaux en un point donné d'une surface. À titre d'exemple, la courbure de Gauss d'une feuille plane ou enroulée en cône ou cylindre est nulle. Elle est positive pour une sphère et négative pour une selle.

Le corps déformable élastiquement 12 est réalisé par exemple en un élastomère présentant un module élastique, mesuré à 23°C selon la norme NFT 46-002, compris entre 10 kPa et 200 MPa.

Dans cet exemple, le corps déformable élastiquement 12 est réalisé par exemple à partir de polyuréthane, de silicone, de latex naturel, ou de tout autre élastomère naturel ou artificiel ou de leurs mélanges.

Dans l'exemple illustré par la figure 5, le corps déformable élastiquement 12 présente une forme de plaque ou de coque dans sa configuration de repos.

Il comporte au moins une région supérieure pleine 15, au moins une région inférieure pleine 16, et entre la région supérieure 15 et la région inférieure 16, une pluralité de parois intermédiaires 18 situées à distance l'une de l'autre.

Dans cet exemple, les parois 18 sont formées par des cloisons qui présentent une étendue longitudinale (leur périmètre sur la figure 4) supérieure à leur hauteur H, prise entre la région supérieure 15 et la région inférieure 16, notamment au moins 10 fois supérieure à leur hauteur H à la périphérie du corps déformable élastiquement 12.

Dans l'exemple représenté sur la figure 4, les parois 18 s'étendent concentriquement à partir d'un axe central A-A' de la structure 10, vers la périphérie 22 de la structure 10.

En référence aux figures 5 et 6, chaque cavité 14 est délimitée, dans au moins une section transversale de la cavité, latéralement par deux parois 18 en regard. Chaque cavité 14 est délimitée au-dessus des parois 18, par la région supérieure 15 et en dessous des parois 18, par la région inférieure 16.

Chaque cavité 14 présente dans cette section un contour fermé, qui est, dans cet exemple particulier, polygonal dans la configuration de repos. La section de la cavité 14 est allongée entre la région supérieure 15 et la région inférieure 16.

Ainsi, dans la configuration de repos, la hauteur H de la cavité 14 est supérieure à la largeur L de la cavité 14, notamment au moins 2 fois supérieure à la largeur L de la cavité 14.

De même, la hauteur H de la cavité 14 est supérieure à deux fois l'épaisseur E1 de la région supérieure 15.

La hauteur H de la cavité 14 est également supérieure à deux fois l'épaisseur E2 de la région inférieure 16, notamment supérieure à 5 fois l'épaisseur E2.

Par ailleurs, la largeur L de la cavité 14 est inférieure à trois fois l'épaisseur EP1 du corps déformable élastiquement 12, prise entre la surface supérieure 14A et la surface inférieure 14B.

Les cavités 14 d'au moins un réseau de cavités 14 sont reliées entre elles. Dans cet exemple, toutes les cavités 14 sont reliées entre elles.

En outre, chaque réseau de cavités 14 reliées entre elles est raccordé à un port 13 pour permettre leur mise en pression sélective.

Le rapport du volume des cavités 14 au volume de matière dans le corps déformable élastiquement 12 est avantageusement inférieur à 50% notamment compris entre 5 et 30%.

En référence à l'exemple particulier représenté sur la figure 1, dans la configuration de repos, le corps déformable élastiquement 12 est plat et forme une plaque. La courbure de Gauss de chaque point de la surface médiane est nulle.

Dans la première configuration mise en pression, représentée sur la figure 2, une pression positive a été introduite dans les cavités 14 du corps déformable élastiquement 12.

Cette pression positive a engendré des contraintes anisotropes au sein du corps déformable élastiquement 12, notamment au niveau des régions 15 et 16. Ces contraintes ont produit une déformation localement faible de la structure des cavités 14, qui engendre une déformation macroscopique importante du corps déformable élastiquement 12 pour engendrer une forme tridimensionnelle dont au moins une partie des points présente une courbure gaussienne non nulle, ici une courbure gaussienne positive à son sommet, et négative sur les bords.

La déformation macroscopique du corps déformable élastiquement 12 a conduit à une variation de métrique sur la surface médiane S.

Selon l'invention, dans chaque configuration mise en pression, le rayon de courbure R de la surface supérieure 14A ou/et de la surface inférieure 14B du corps déformable élastiquement 12, défini comme le minimum des deux rayons de courbure principaux de la surface 14A, 14B, pris en regard de chaque cavité interne 14 adjacente à la surface 14A, 14B, est supérieur à deux fois la taille de la cavité 14 adjacente à la surface 14A, 14B .

La taille T de la cavité 14 est définie comme le diamètre de la plus grosse sphère qui peut être insérée dans la cavité 14 sans déformation.

De même, la largeur L de chaque cavité interne 14 adjacente à la surface extérieure 14A, 14B, est inférieure à deux fois la distance DS de la cavité interne 14 à la surface extérieure 14A, 14B du corps déformable élastiquement 12 la plus proche de la cavité interne 14.

Pour déterminer la distance DS pour chaque cavité interne 14, on définit le segment le plus court qui relie la surface interne de la cavité et la surface externe du corps déformable élastiquement, la distance DS étant la longueur de ce segment. La largeur L de la cavité est définie comme la plus petite des largeurs de la cavité interne 14 mesurées perpendiculairement au segment précité.

Par « cavité adjacente à la surface », on entend ici une cavité dont le segment DS qui les relie à la surface ne traverse pas d'autre cavité.

Les propriétés précitées sont vérifiées notamment lorsque la pression appliquée dans la configuration mise en pression est l'opposé de la pression atmosphérique et également lorsque la pression appliquée dans la configuration mise en pression est quatre fois le module élastique du matériau formant le corps déformable élastiquement.

Dans cet exemple où la structure 10 est en forme de plaque, le rayon de courbure macroscopique RC d'au moins une région du corps déformable élastiquement 12 comprenant des cavités 14 a diminué pour présenter une valeur non infinie, toutefois supérieure à trois fois l'épaisseur du corps déformable élastiquement 12.

Le rayon de courbure macroscopique minimal au sein de la structure est notamment inférieur à 10 cm et est par exemple compris entre 1,5 cm et 2 m. Le rayon de courbure macroscopique est défini comme le minimum des deux rayons de courbure principaux locaux de la surface médiane S dans la configuration mise en pression.

Au contraire, la déflexion locale DL de la région supérieure 15 du corps 12 (voir figure 6) et de la région inférieure 16 du corps 12 en regard de chaque cavité 14 est restée limitée.

Ainsi, la déflexion locale DL du corps déformable élastiquement 12 en regard de chaque cavité interne 14 dans chaque configuration mise en pression est inférieure à au moins 5 fois le rayon de courbure macroscopique du corps, pris en regard de la cavité interne.

La déformation locale DL est déterminée respectivement sur la surface supérieure 14A et sur la surface inférieure 14B, comme la hauteur locale de chaque bosse se formant en regard de chaque cavité 14.

La variation maximale de l'aire de la section de la cavité 14 est inférieure à 400% entre l'aire de la section SE2 de la cavité 14 dans la configuration mise en pression et l'aire de la section SE1 de la cavité 14 dans la configuration mise en pression . Ceci assure une déformation macroscopique du corps 12, en minimisant la déformation au niveau de chaque cavité 14.

La variation maximale d'épaisseur du corps 12, prise entre l'épaisseur EP2 mesurée dans la configuration mise en pression et l'épaisseur EP1 mesurée dans la configuration de repos est inférieure à 100 % et est notamment comprise entre 5 % et 50 %.

Ainsi, une forme souhaitée et préprogrammée de la structure 10 est obtenue simplement et rapidement dans chaque configuration mise en pression, sans que l'aspect extérieur de la structure 10 ne soit détérioré, puisque l'expansion hors plan des cavités 14 est très limitée au sein du corps 12 pour favoriser la déformation macroscopique dans le plan de la structure 10 et l'obtention d'une variation de métrique macroscopique.

Dans l'exemple représenté sur la figure 2, le corps déformable élastiquement 12 présente ainsi une forme de dôme dans la configuration mise en pression, avec une pression positive.

Comme illustré sur la figure 3, dans une deuxième configuration mise en pression lorsqu'une pression négative est appliquée à l'intérieur des cavités 14 du corps 12, au moins une partie des points de la surface médiane présente une courbure de Gauss négative, et le corps déformable élastiquement 12 occupe alors une forme de selle de cheval.

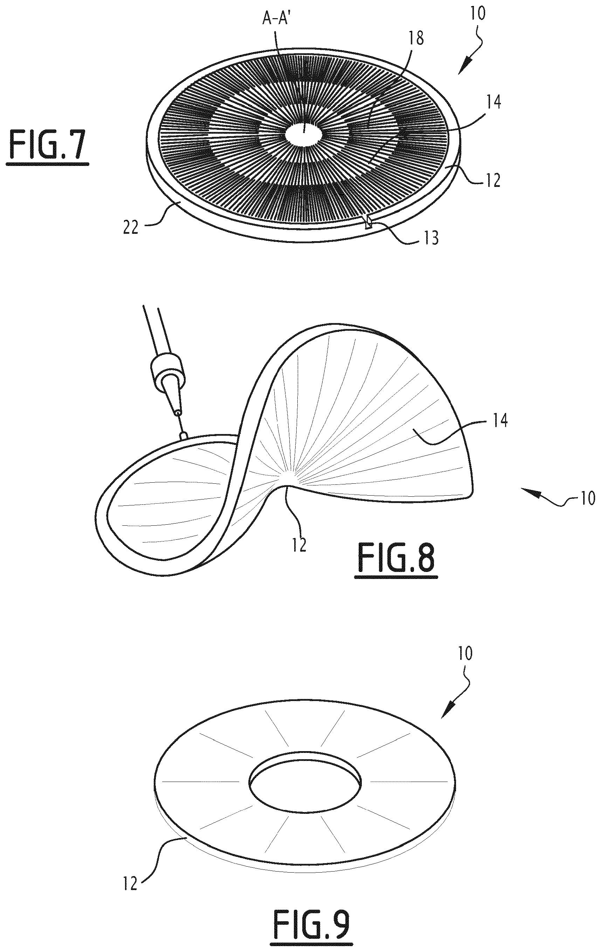

Dans la variante représentée sur les figures 7 et 8, les parois 18 délimitant latéralement les cavités 14 s'étendent radialement depuis un axe central A-A' du corps déformable élastiquement 12 vers la périphérie 22.

À l'inverse de la structure 10 représentée sur la figure 4, l'application d'une pression positive pour passer dans la première configuration mise en pression conduit le corps déformable élastiquement 12 à adopter une forme de selle de cheval, comme illustré sur la figure 8. Au contraire, l'application d'une pression négative pour passer dans la deuxième configuration mise en pression conduit le corps déformable élastiquement 12 à adopter une forme de dôme.

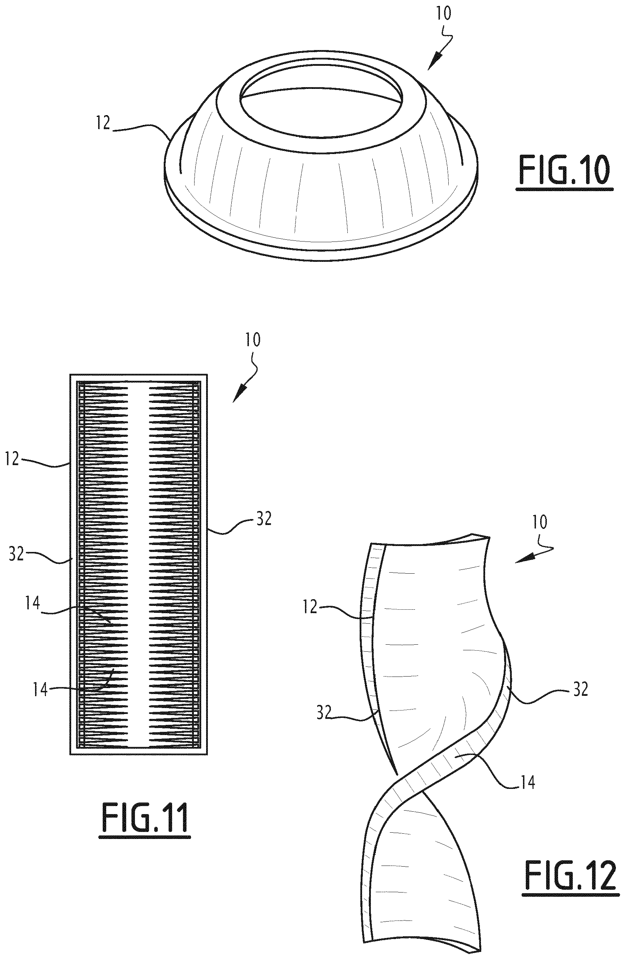

Dans la variante représentée sur la figure 9, un orifice central 30 traversant est ménagé au centre du corps déformable élastiquement 12. Dans la configuration mise en pression représentée sur la figure 10, le corps déformable élastiquement 12 adopte ainsi une forme de dôme tronqué.

Dans la variante représentée sur la figure 11, le corps déformable élastiquement 12 présente une forme sensiblement rectangulaire dans la configuration de repos. Des cavités 14 sont ménagées dans les régions latérales 32 du corps déformable élastiquement 12, perpendiculairement à un axe longitudinal B-B' du corps déformable élastiquement 12. Au contraire, la région centrale 34 du corps déformable élastiquement 12 est dépourvue de cavités 14.

Comme illustré par la figure 12, l'application d'une pression dans les cavités 14 engendre une déformation par torsion des régions latérales 32 du corps déformable élastiquement 12, la torsion étant dans un sens le long d'un premier bord 32 et dans un sens opposé le long d'un bord opposé du corps déformable élastiquement 12.

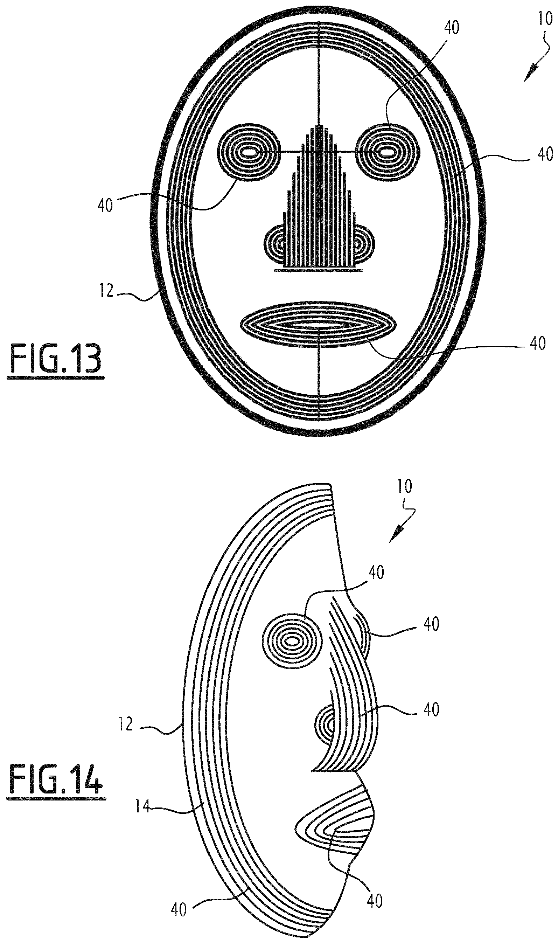

Dans la variante représentée sur les figures 13 et 14, le corps déformable élastiquement 12 présente des régions choisies 40 munies de cavités 14, chaque région 40 étant propre à engendrer une forme particulière, et des régions 42 dépourvues de cavités 14.

Par exemple, le corps déformable élastiquement 12 comprend une région périphérique 40 munie de cavités, et quatre régions centrales 40 munies de cavités, propre à former ici respectivement la bouche, le nez, et des yeux d'une face humaine dans la configuration mise en pression.

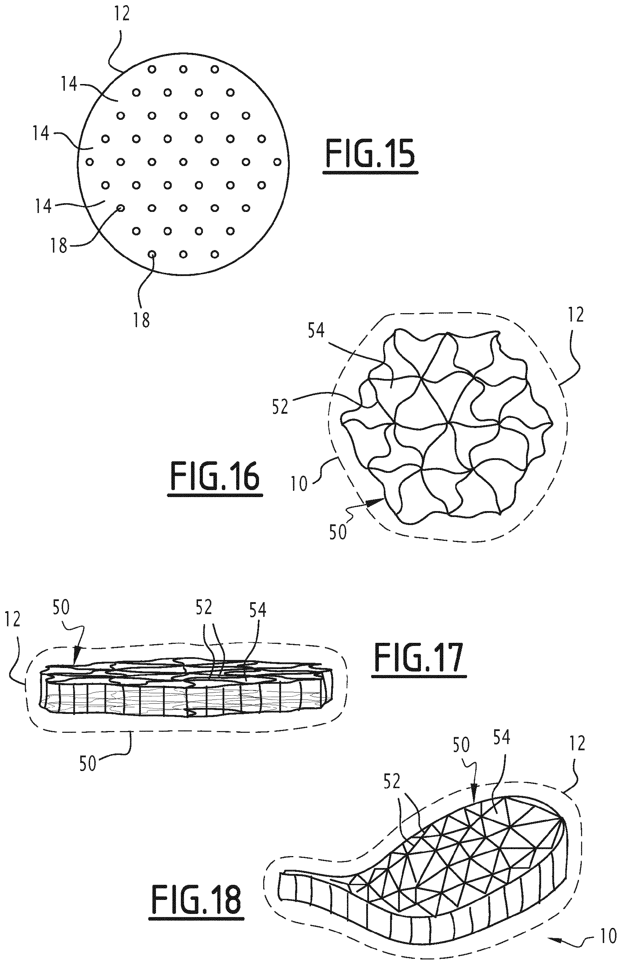

Dans la variante illustrée par la figure 15, les parois latérales 18 délimitant chaque cavité 14 sont formées par des piliers. L'étendue transversale des piliers est généralement inférieure à 2 fois leur hauteur.

Le fonctionnement de la structure 10 décrite sur la figure 15 est analogue au fonctionnement des structures 10 décrites sur les figures précédentes.

Les structures 10 selon l'invention sont donc facilement déformables, et permettent d'atteindre des formes prédéterminées par la structure des cavités 14 contenues dans le corps déformable élastiquement 12.

Cette déformation est très rapide, permettant de passer simplement et de manière quasi immédiate de la configuration de repos à la configuration mise en pression, ou d'une première configuration mise en pression à une deuxième configuration mise en pression.

Pour fabriquer des structures 10 précitées, présentant une forme souhaitée dans la configuration mise en pression, il est possible de modéliser la forme et la métrique de la structure 10, et la position des cavités 14 nécessaires pour obtenir cette forme et cette métrique souhaitée. Ensuite, la forme et la métrique correspondante de la structure 10 et des cavités 14 dans la configuration de repos sont calculées pour obtenir la position des parois 18 à former par moulage.

Un moule de forme complémentaire à la forme calculée est fabriqué et la région inférieure 16 et les parois 18 sont formées par moulage.

Puis, la région supérieure 15 est formée ou est assemblée sur les parois 18 pour fermer les cavités 14 et rendre les cavités 14 étanches.

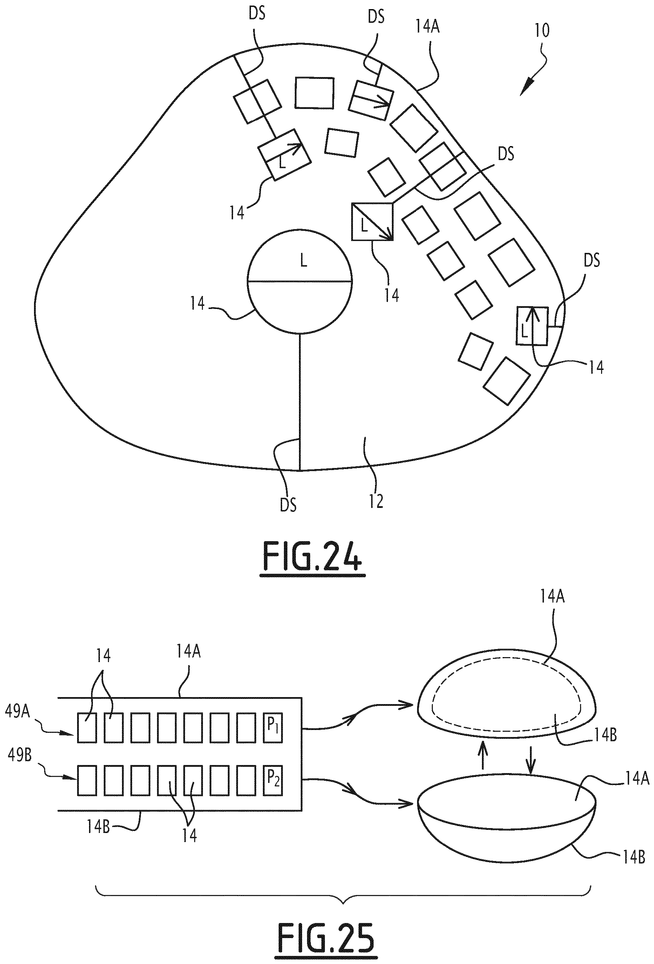

Dans une variante représentée sur la figure 24, le corps déformable élastiquement 12 présente une forme volumique tridimensionnelle dans sa configuration de repos.

Comme précédemment, dans chaque configuration mise en pression, le rayon de courbure R de la surface extérieure 14A du corps déformable élastiquement 12, défini comme le minimum des deux rayons de courbure principaux de la surface extérieure 14A, pris en regard de chaque cavité interne 14 adjacente à la surface extérieure 14A, est supérieur à deux fois la taille de la cavité 14 adjacente à la surface extérieure 14A.

De même, la largeur L de chaque cavité interne 14 adjacente à la surface extérieure 14A est inférieure à trois fois la distance DS de la cavité interne 14 à la surface extérieure 14A du corps déformable élastiquement 12 la plus proche de la cavité interne 14 adjacente à la surface extérieure 14A, la distance DS et la largeur L étant définies comme précédemment.

Dans la variante représentée sur la figure 25, le corps déformable élastiquement 12 comporte au moins deux réseaux 49A, 49B de cavités internes 14 disposés de manière étagée l'un sur l'autre. Les réseaux 49A, 49B sont propres à être mis en pression de manière indépendante l'un de l'autre.

Comme illustré sur la figure 25, lorsque le premier réseau 49A de cavités 14 est mis sous pression, le corps 12 se déforme suivant une première convexité orientée dans un premier sens, dans laquelle la surface 14A est convexe et la surface 14B est concave. Au contraire, lorsque le deuxième réseau 49B de cavités 14 est mis sous pression, le corps 12 se déforme suivant une deuxième convexité dans un deuxième sens opposé au premier sens, dans lequel la surface 14A est concave et la surface 14B est convexe.

Dans la variante représentée sur les figures 16 à 18, la structure 10 comporte en outre une armature interne 50 noyée dans le corps déformable élastiquement 12. L'armature interne 50 est déployable entre un état contracté, visible sur les figures 16 et 17 et un état déployé, visible sur la figure 18.

Dans cet exemple, l'armature 50 est formée d'un réseau de fils 52 définissant des mailles 54 liées entre elles. L'épaisseur des fils 52 est inférieure à au moins 10 fois leur longueur.

Les fils 52 présentent un module élastique, mesuré par la méthode décrite plus haut, supérieur au module élastique du corps déformable élastiquement 12. Ce module élastique est par exemple supérieur à 10GPa et compris notamment entre 0,1 GPa et 1000 GPa. Ainsi, les fils 52 sont quasi inextensibles.

Dans l'état contracté, visible sur la figure 16, les fils 52 sont lâches et chaque maille 54 présente une aire minimale. Dans l'exemple représenté sur la figure 17, l'armature 50 présente une forme plane correspondant à la configuration de repos du corps déformable élastiquement 12.

Dans l'état déployé, visible sur la figure 18, les fils 52 sont tendus. Chaque maille 54 présente une aire maximale.

L'armature 50 présente alors une structure tridimensionnelle rigide correspondant à la configuration mise en pression du corps déformable élastiquement 12. L'armature 50 limite ainsi l'expansion du corps déformable élastiquement 12 dans cette configuration mise en pression.

Le corps déformable élastiquement 12 est rigidifié par des fils 52 tendus de l'armature 50 présentant un module élastique supérieur à celui du corps déformable élastiquement 12. Cette rigidification structurelle du corps déformable élastiquement 12 s'effectue par effet de tenségrité.

Avantageusement, le corps 12 n'est plus déformable au toucher dans la configuration mise en pression.

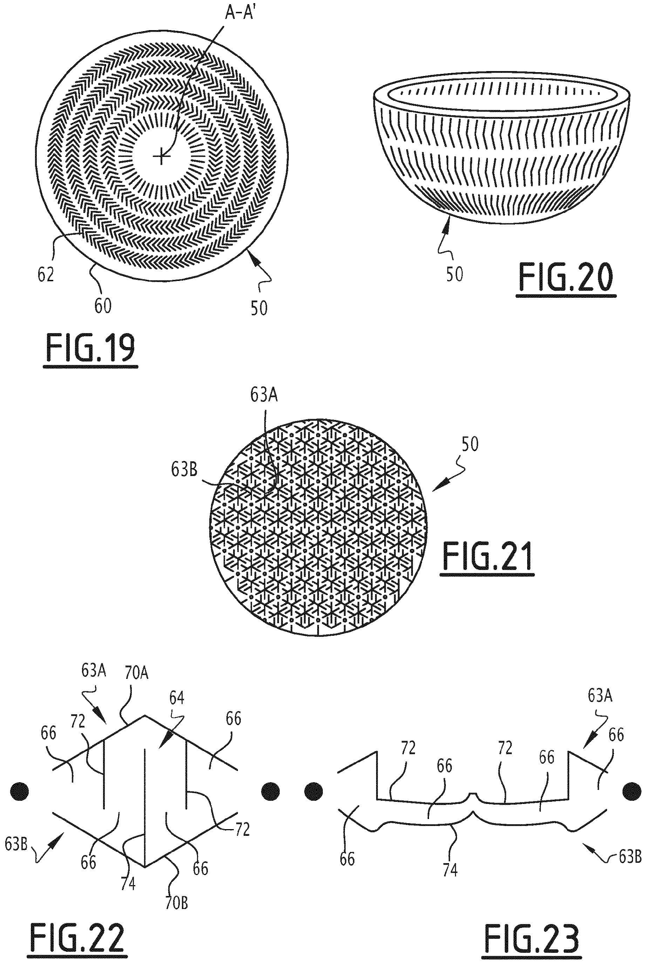

Dans une variante représentée sur la figure 19, l'armature interne 50 comporte au moins une feuille 60 d'un matériau inextensible découpé en des motifs 62 de lamelles. Dans l'exemple représenté sur la figure 19, les motifs de découpe 62 présentent des formes de chevrons. Les chevrons sont ici disposés concentriquement autour de l'axe central A-A'

La feuille 60 est noyée dans le corps déformable élastiquement 12.

Dans l'état contracté, la feuille 60 est plane et correspond à la configuration de repos du corps déformable élastiquement 12. Les branches de chaque chevron constituant un motif 62 sont rapprochées l'une de l'autre.

Dans l'état déployé, visible sur la figure 20, la feuille 60 s'est déformée grâce aux découpes 62 pour occuper un état déployé tridimensionnel. Les motifs 62 se sont allongés, par écartement des branches de chaque chevron constituant un motif 62. Cet allongement produit une rigidification de la feuille 60 et par suite, du corps 62 par effet de tenségrité.

Dans une variante, la structure 10 comporte une pluralité de feuilles 60 étagées dans le corps déformable élastiquement 12.

Dans l'exemple représenté sur les figures 21 à 23, les découpes 62 présentent des formes de fourche 63A, 63B imbriquées les unes dans les autres. Les fourches 63A, 63B en regard définissent entre elles un chemin de découpe sinueux 64 comportant des doigts imbriqués 66 propres à passer d'une configuration parallèle les uns aux autres dans l'état contracté de l'armature 50 (voir figure 22) à une configuration sensiblement alignée les uns avec les autres dans l'état déployé de l'armature 50 (voir figure 23).

Dans l'exemple représenté sur la figure 22, la fourche 63A comporte un chevron 70A s'ouvrant en regard d'un autre chevron symétrique 70B de la fourche 63B. La fourche 63A comporte en outre, entre les branches du chevron 70A, deux traits de découpe parallèles 72 dirigés vers l'autre chevron 70B. La fourche 63B comporte entre les branches du chevron symétrique 70B, un trait de découpe centrale 74 intercalé entre les traits de découpe parallèles 72.

Le chemin de découpe sinueux 64 est délimité entre les chevrons 70A, 70B, en suivant les traits de découpes 72, 74.

Les découpes 62 sont présentes sous forme de motifs répétés dans la feuille 60.

Comme décrit précédemment, la feuille 60 est propre à passer d'un état contracté, dans lequel les doigts imbriqués 66 sont parallèles les uns aux autres, correspondant à la configuration de repos du corps déformable élastiquement 12 à un état déployé, dans laquelle les doigts imbriqués 66 sont alignés les uns avec les autres, rigidifiant la structure 10 dans une configuration mise en pression.

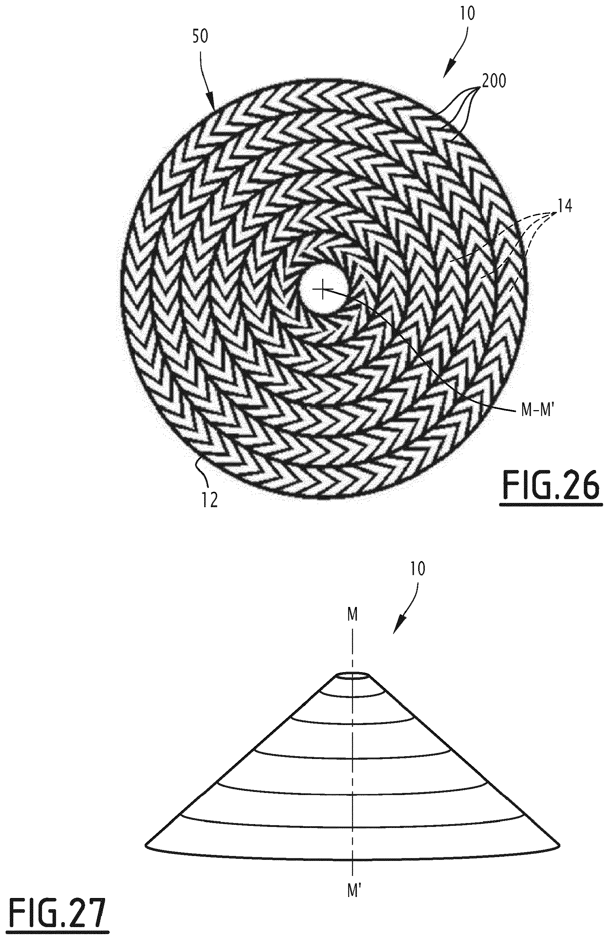

Dans la variante de structure 10 illustrée sur les figures 26 et 27, l'armature interne 50 comporte avantageusement une succession de chevrons 200 répartis circonférentiellement.

Le corps 12 est déformable élastiquement entre une configuration de repos, avantageusement plane et au moins une configuration mise en pression, qui est ici en forme de tronc de cône, comme visible sur la figure 27. Le déploiement des chevrons produit une rigidification de l'armature 50 par effet de tenségrité.

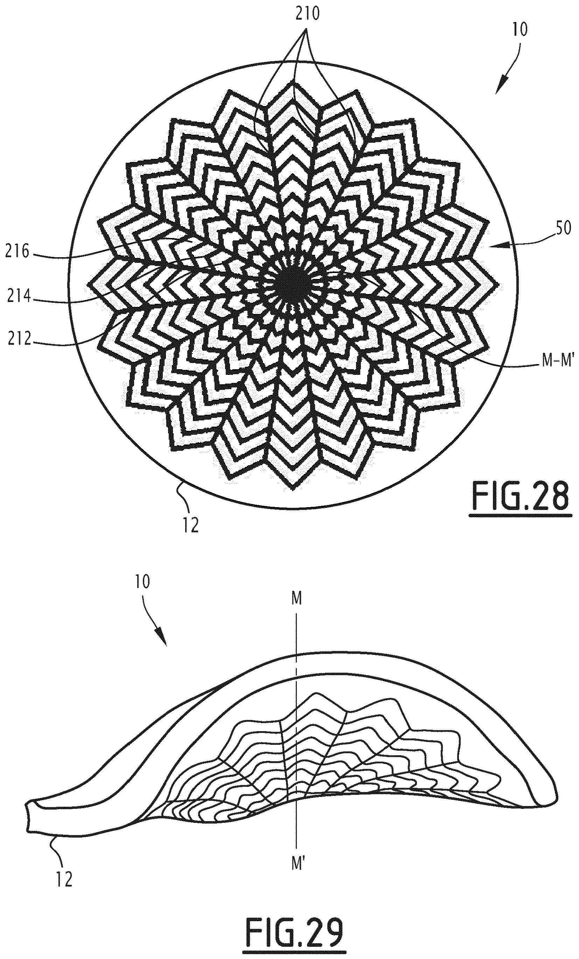

Dans la variante de structure 10 illustrée sur les figures 28 et 29, l'armature interne 50 présente une forme générale de flocon, avec des branches radiales 210 qui divergent radialement à partir d'un axe central M-M'. Les branches radiales 210 sont raccordées entre elles par des chevrons 212, 214, 216 s'étendant sur des circonférences successives autour de l'axe central M-M', de plus en plus éloignées de l'axe central M-M'. La largeur des chevrons 212, 214, 216 croit depuis l'axe central M-M' vers la périphérie de l'armature 50.

Dans cet exemple, la pointe des chevrons 212, 214, 216 pointe à l'écart de l'axe central M-M'.

Le corps 12 est déformable élastiquement entre une configuration de repos, avantageusement plane et au moins une configuration mise en pression, qui est ici en forme de en forme de selle de cheval, comme visible sur la figure 29. Le déploiement des chevrons produit une rigidification de l'armature 50 par effet de tenségrité.

Dans une variante des modes de réalisation décrits précédemment, la structure 10 comporte une couche fine de matériau rigide ciselé, choisi parmi le bois, le plastique, le métal, le linoléum.

L'épaisseur de la couche fine est inférieure à au moins 10 fois l'épaisseur du corps déformable élastiquement 12. Le matériau rigide ciselé se déforme à la surface du corps déformable élastiquement 12 en suivant les déformations du corps déformable élastiquement 12.

Dans une application de la structure 10, les formes et les dimensions du corps déformable élastiquement 12 dans la configuration de repos et dans chacune des configurations mise en pression sont choisies par exemple pour que la structure 10 forme un équipement biomédical, notamment une endoprothèse déployable, ou un actionneur de manipulation et de déplacement de tissu. Dans ce cas, le matériau du corps 12 est biocompatible.

En variante, la structure 10 forme un équipement de sport et de rééducation. Par exemple, la structure 10 forme un dispositif d'assistance de forme réglable pour la musculation ou la rééducation. En variante, la structure 10 forme une structure gonflable déployable pour la haute couture, l'aérospatial ou pour une activité extérieure, telle que des tentes, de bols ou autres accessoires.

Dans encore une variante, la structure 10 forme une partie d'un meuble, par exemple un panneau de meuble continûment déformable ou une charnière activable pneumatiquement.

Dans encore une autre variante, la structure 10 forme un équipement industriel, présentant une forme adaptée suivant l'usage, par exemple un appui-tête déformable qui épouse la forme du crâne, ou une pale d'éolienne de forme variable pour optimiser le rendement.

Selon l'invention, dans chaque configuration mise en pression, le rayon de courbure d'une surface extérieure du corps déformable élastiquement 12, pris en regard de chaque cavité interne 14 adjacente à la surface extérieure, est supérieur à deux fois la taille de la cavité interne 14 adjacente à la surface extérieure. Cette propriété peut s'appliquer à toutes les cavités internes 14 qu'elles soient ou non adjacentes à la surface extérieure ou au contraire peut ne pas s'appliquer à certaines des cavités non adjacentes à la surface extérieure.

## Revendications

1. Structure pneumatique (10) comportant un corps déformable élastiquement (12) définissant au moins un réseau de cavités internes (14), chaque cavité interne (14) présentant un contour fermé dans au moins une section de la cavité interne (14),
chaque cavité interne (14) étant propre à être mise en pression pour faire passer le corps déformable élastiquement (12) d'une configuration de repos à au moins une configuration mise en pression,
**caractérisée en ce que** dans chaque configuration mise en pression, le corps déformable élastiquement (12) présente une métrique macroscopique distincte de sa métrique macroscopique en configuration de repos,
et **en ce que**, dans chaque configuration mise en pression, le rayon de courbure d'une surface extérieure du corps déformable élastiquement (12), pris en regard de chaque cavité interne (14) adjacente à la surface extérieure, est supérieur à deux fois la taille de la cavité interne (14) adjacente à la surface extérieure.

2. Structure (10) selon la revendication 1, dans laquelle, dans chaque configuration mise en pression, la largeur de chaque cavité interne (14), est inférieure à trois fois, notamment inférieure à deux fois, la distance de la cavité interne (14) à la surface extérieure du corps déformable élastiquement (12).

3. Structure (10) selon l'une quelconque des revendications précédentes, dans laquelle chaque cavité interne (14) est délimitée latéralement, dans ladite section, par deux parois latérales (18), par une région inférieure (16) et par une région supérieure (15) du corps déformable élastiquement (12).

4. Structure (10) selon la revendication 3, dans laquelle dans au moins une section, la largeur de la cavité interne (14), prise entre les parois latérales (18) est inférieure à trois fois l'épaisseur du corps déformable élastiquement (12).

5. Structure (10) selon l'une quelconque des revendications 3 à 4, dans laquelle dans au moins une section, l'épaisseur de la région supérieure (15) ou/et l'épaisseur de la région inférieure (16), prise en regard de la cavité interne (14) est supérieure à la moitié de la largeur de la cavité interne (14), prise entre les parois latérales (18).

6. Structure (10) selon l'une quelconque des revendications 3 à 5, comprenant une pluralité de cavités internes (14) adjacentes séparées entre elles par des parois latérales (18) de longueur supérieure à la largeur de la cavité interne (14), les parois latérales (18) s'étendant avantageusement radialement par rapport à un axe central, ou circonferentiellement autour d'un axe central.

7. Structure (10) selon l'une quelconque des revendications 3 à 6, comprenant une pluralité de cavités internes (14) adjacentes séparées entre elles par des piliers, la distance entre les surfaces externes des piliers est inférieure à deux fois l'épaisseur de la région supérieure et inférieure.

8. Structure (10) selon l'une quelconque des revendications précédentes, dans laquelle au moins une cavité interne (14) du corps déformable élastiquement (12) est propre à recevoir une pression comprise entre l'opposé de la pression atmosphérique et quatre fois le module élastique du corps déformable élastiquement (12), le corps déformable élastiquement (12) présentant une première métrique macroscopique lorsque la pression appliquée est l'opposé de la pression atmosphérique et une deuxième métrique macroscopique distincte de la première métrique macroscopique lorsque la pression appliquée est quatre fois le module élastique.

9. Structure (10) selon l'une quelconque des revendications précédentes, dans laquelle chaque cavité interne (14) dans chaque configuration mise en pression présente une section d'aire inférieure à 400% de l'aire de la section de la cavité interne (14) dans la configuration de repos.

10. Structure (10) selon l'une quelconque des revendications précédentes, comprenant une armature interne (50) déployable entre un état contracté et un état déployé lors du passage du corps déformable élastiquement (12) de la configuration de repos à la configuration mise en pression, l'armature interne (50) présentant dans l'état déployé un module élastique supérieur à celui du corps déformable élastiquement (12) pour rigidifier le corps déformable élastiquement (12), l'épaisseur de l'armature interne (50) étant avantageusement au moins 10 fois plus petite que la plus grande dimension de l'armature interne (50).

11. Structure (10) selon la revendication 10, dans laquelle l'armature interne (50) comporte un réseau de fils (52) non tendus dans l'état contracté et tendus dans l'état déployé, ou au moins un étage de feuille (60) inextensible découpée, en particulier par des motifs en forme de fourche imbriqués.

12. Structure (10) selon l'une quelconque des revendications précédentes, comprenant un premier réseau de cavités internes (14) propre à être mis en pression et au moins un deuxième réseau de cavités internes (14) propre à être mis en pression indépendamment du premier réseau de cavités internes (14), le premier réseau de cavités internes (14) et le deuxième réseau de cavités internes (14) étant avantageusement étagés.

13. Structure (10) selon l'une quelconque des revendications précédentes, formant un équipement biomédical, un équipement de loisir et/ou de rééducation, un composant de meuble, un équipement industriel de forme adaptable, notamment un appui tête réglable ou une pale d'éolienne.

14. Procédé de fabrication d'une structure (10) selon l'une quelconque des revendications précédentes, comprenant les étapes suivantes :
- moulage d'une première partie du corps déformable élastiquement (12) présentant chaque cavité interne (14), chaque cavité interne (14) étant ouverte ;
- fermeture de chaque cavité interne (14) par application d'une deuxième partie du corps déformable élastiquement (12) sur la première partie du corps déformable élastiquement (12).

15. Procédé selon la revendication 14, comprenant, lors du moulage, l'incorporation d'une armature interne (50) déployable entre un état contracté et un état déployé lors du passage du corps déformable élastiquement (12) de la configuration de repos à la configuration mise en pression, l'armature interne (50) dans l'état déployé verrouillant le corps déformable élastiquement (12) dans la configuration mise en pression.

## Patentansprüche

1. Pneumatische Struktur (10), umfassend einen elastisch verformbaren Körper (12), der mindestens ein Netz von inneren Hohlräumen (14) definiert, wobei jeder innere Hohlraum (14) eine geschlossene Kontur in mindestens einem Querschnitt des inneren Hohlraums (14) aufweist,
wobei jeder innere Hohlraum (14) geeignet ist, um unter Druck gesetzt zu werden, um den elastisch verformbaren Körper (12) von einer Ruhekonfiguration in mindestens eine druckbeaufschlagte Konfiguration zu überführen,
**dadurch gekennzeichnet, dass** der elastisch verformbare Körper (12) in jeder druckbeaufschlagten Konfiguration eine makroskopische Metrik aufweist, die sich von seiner makroskopischen Metrik in der Ruhekonfiguration unterscheidet,
und dass in jeder druckbeaufschlagten Konfiguration der Krümmungsradius einer Außenfläche des elastisch verformbaren Körpers (12) gegenüber jedem inneren Hohlraum (14) angrenzend an die Außenfläche größer ist als die zweifache Größe des inneren Hohlraums (14), der an die Außenfläche angrenzt.

2. Struktur (10) nach Anspruch 1, wobei in jeder druckbeaufschlagten Konfiguration die Breite von jedem inneren Hohlraums (14) weniger als das Dreifache, insbesondere weniger als das Zweifache, des Abstands des inneren Hohlraums (14) von der Außenfläche des elastisch verformbaren Körpers (12) ist.

3. Struktur (10) nach einem der vorherigen Ansprüche, wobei jeder innere Hohlraum (14) in diesem Querschnitt seitlich durch zwei Seitenwände (18), durch einen unteren Bereich (16) und durch einen oberen Bereich (15) des elastisch verformbaren Körpers (12) begrenzt ist.

4. Struktur (10) nach Anspruch 3, wobei die Breite des inneren Hohlraums (14), genommen zwischen den Seitenwänden (18), in mindestens einem Querschnitt weniger als dreimal so groß ist wie die Stärke des elastisch verformbaren Körpers (12)

5. Struktur (10) nach einem der Ansprüche 3 bis 4, wobei die Stärke des oberen Bereichs (15) oder/und die Stärke des unteren Bereichs (16), genommen in Bezug auf den inneren Hohlraum (14), in mindestens einem Querschnitt größer ist als die Hälfte der Breite des inneren Hohlraums (14), genommen zwischen den Seitenwänden (18).

6. Struktur (10) nach einem der Ansprüche 3 bis 5, umfassend eine Vielzahl von angrenzenden inneren Hohlräumen (14), die durch Seitenwände (18) untereinander getrennt sind, deren Länge größer ist als die Breite des inneren Hohlraums (14), wobei sich die Seitenwände (18) vorteilhafterweise radial zu einer Mittelachse oder im Umfang um eine Mittelachse erstrecken.

7. Struktur (10) nach einem der Ansprüche 3 bis 6, umfassend eine Vielzahl von angrenzenden inneren Hohlräumen (14), die durch Pfeiler untereinander getrennt sind, wobei der Abstand zwischen den Außenflächen der Pfeiler weniger als das Doppelte der Stärke des oberen und unteren Bereichs ist.

8. Struktur (10) nach einem der vorherigen Ansprüche, wobei mindestens ein innerer Hohlraum (14) des elastisch verformbaren Körpers (12) geeignet ist, um einen Druck zu erhalten, der zwischen dem Gegenteil des atmosphärischen Drucks und dem Vierfachen des Elastizitätsmoduls des elastisch verformbaren Körpers (12) liegt, wobei der elastisch verformbare Körper (12) eine erste makroskopische Metrik aufweist, wenn der angelegte Druck das Gegenteil des Atmosphärendrucks ist, und eine zweite makroskopische Metrik, die sich von der ersten makroskopischen Metrik unterscheidet, wenn der angelegte Druck das Vierfache des Elastizitätsmoduls ist.

9. Struktur (10) nach einem der vorherigen Ansprüche, wobei jeder innere Hohlraum (14) in jeder druckbeaufschlagten Konfiguration eine Querschnittsfläche aufweist, die kleiner ist als 400 % der Querschnittsfläche des inneren Hohlraums (14) in der Ruhekonfiguration.

10. Struktur (10) nach einem der vorherigen Ansprüche, umfassend eine innere Verstärkung (50), die bei Übergang des elastisch verformbaren Körpers (12)aus der Ruhekonfiguration in die druckbeaufschlagte Konfiguration zwischen einem zusammengezogenen Zustand und einem entfalteten Zustand entfaltbar ist, wobei die innere Verstärkung (50) in dem entfalteten Zustand einen höheren Elastizitätsmodul aufweist als der elastisch verformbare Körper (12), um den elastisch verformbaren Körper (12) zu versteifen, wobei die Stärke der inneren Verstärkung (50) vorteilhafterweise mindestens 10 Mal kleiner ist als die größte Abmessung der inneren Verstärkung (50).

11. Struktur (10) nach Anspruch 10, wobei die innere Verstärkung (50) ein Netz aus Fäden (52) umfasst, die in dem zusammengezogenen Zustand nicht gespannt sind und in dem entfalteten Zustand gespannt sind, oder mindestens eine Lage aus einer undehnbaren Folie (60), die insbesondere durch verschachtelte gabelförmige Muster geschnitten ist.

12. Struktur (10) nach einem der vorherigen Ansprüche, umfassend ein erstes Netz aus inneren Hohlräumen (14), das geeignet ist, um druckbeaufschlagt zu werden, und mindestens ein zweites Netz aus inneren Hohlräumen (14), das geeignet ist, um unabhängig von dem ersten Netz aus inneren Hohlräumen (14) druckbeaufschlagt zu werden, wobei das erste Netz aus inneren Hohlräumen (14) und das zweite Netz aus inneren Hohlräumen (14) vorteilhafterweise gestaffelt sind.

13. Struktur (10) nach einem der vorherigen Ansprüche, die eine biomedizinische Ausrüstung, eine Freizeit- und/oder Rehabilitationsausrüstung, eine Möbelkomponente, eine industrielle Ausrüstung mit anpassungsfähiger Form, insbesondere eine verstellbare Kopfstütze oder ein Windradblatt, bildet.

14. Verfahren zur Herstellung einer Struktur (10) nach einem der vorherigen Ansprüche, umfassend die folgenden Schritte:
- Gießen eines ersten Abschnitts des elastisch verformbaren Körpers (12), der jeden inneren Hohlraum (14) aufweist, wobei jeder innere Hohlraum (14) offen ist;
- Schließen jedes inneren Hohlraums (14) durch Anlegen eines zweiten Abschnitts des elastisch verformbaren Körpers (12) an den ersten Abschnitt des elastisch verformbaren Körpers (12).

15. Verfahren nach Anspruch 14, umfassend, beim Gießen, das Integrieren einer inneren Verstärkung (50), die bei Übergang des elastisch verformbaren Körpers (12) aus der Ruhekonfiguration in die druckbeaufschlagte Konfiguration zwischen einem zusammengezogenen Zustand und einem entfalteten Zustand entfaltbar ist, wobei die innere Verstärkung (50) in dem entfalteten Zustand den elastisch verformbaren Körper (12) in der druckbeaufschlagten Konfiguration verriegelt.

## Claims

1. A pneumatic structure (10) comprising an elastically deformable body (12) defining at least one network of internal cavities (14), each internal cavity (14) having a closed contour in at least one section of the internal cavity (14),
each internal cavity (14) being capable of being pressurized so as to cause the elastically deformable body (12) to pass from a rest configuration to at least one pressurized configuration,
**characterized in that** in each pressurized configuration, the elastically deformable body (12) has a macroscopic metric that is distinct from its macroscopic metric in the rest configuration;
and **in that**, in each pressurized configuration, the radius of curvature of an exterior surface of the elastically deformable body (12), taken opposite each internal cavity (14) adjacent to the exterior surface, is greater than two times the size of the internal cavity (14) adjacent to the exterior surface.

2. A structure (10) according to claim 1, in which, in each pressurized configuration, the width of each internal cavity (14) is less than three times, in particular less than two times, the distance from the internal cavity (14) to the exterior surface of the elastically deformable body (12).

3. A structure (10) according to any one of the preceding claims, in which each internal cavity (14) is delimited laterally, in the said section, by two lateral walls (18), by a lower region (16) and by a upper region (15) of the elastically deformable body (12)

4. A structure (10) according to claim 3, in which in at least one section, the width of the internal cavity (14), taken between the lateral walls (18) is less than three times the thickness of the elastically deformable body (12).

5. A structure (10) according to any one of claims 3 to 4, in which in at least one section, the thickness of the upper region (15) or/and the thickness of the lower region (16), taken opposite the internal cavity (14) is greater than half the width of the internal cavity (14), taken between the lateral walls (18).

6. A structure (10) according to any one of claims 3 to 5, comprising a plurality of adjacent internal cavities (14) separated from one another by lateral walls (18) the length thereof is greater than the width of the internal cavity (14), the lateral walls (18) advantageously extending radially in relation to a central axis, or circumferentially around a central axis.

7. A structure (10) according to any one of claims 3 to 6, comprising a plurality of adjacent internal cavities (14) separated from one another by pillars, the distance between the exterior surfaces of the pillars being less than two times the thickness of the upper and lower region.

8. A structure (10) according to any one of the preceding claims, in which at least one internal cavity (14) of the elastically deformable body (12) is capable of receiving a pressure in a range between the opposite of atmospheric pressure and four times the elastic modulus of the elastically deformable body (12), the elastically deformable body (12) having a first macroscopic metric when the pressure applied is the opposite of atmospheric pressure, and a second macroscopic metric that is distinct from the first macroscopic metric when the pressure applied is four times the elastic modulus.

9. A structure (10) according to any one of the preceding claims, in which each internal cavity (14) in each pressurized configuration has a section with an area that is less than 400% of the area of the section of the internal cavity (14) in the rest configuration.

10. A structure (10) according to any one of the preceding claims, comprising an internal reinforcing framework (50) deployable between a contracted state and a deployed state during the transition of the elastically deformable body (12) passing from the rest configuration to the pressurized configuration, the internal reinforcing framework (50) having in the deployed state an elastic modulus that is greater than that of the elastically deformable body (12) in order to stiffen the elastically deformable body (12), the thickness of the internal reinforcing framework (50) being advantageously at least 10 times smaller than the greatest dimension of the internal reinforcing framework (50).

11. A structure (10) according to claim 10, in which the internal reinforcing framework (50) includes a network of wires (52) that is unstretched in the contracted state and stretched in the deployed state, or at least one tier of inextensible sheeting (60) cutout, in particular with interlocking fork-shaped patterns.

12. A structure (10) according to any one of the preceding claims, comprising a first network of internal cavities (14) that is capable of being pressurized, and at least a second network of internal cavities (14) that is capable of being pressurized independently of the first network of internal cavities (14), the first network of internal cavities (14) and the second network of internal cavities (14) being advantageously tiered.

13. A structure (10) according to any one of the preceding claims, that forms a biomedical equipment item/unit, a recreational and/or rehabilitation equipment item/unit, a furniture component, an industrial equipment item/unit having an adaptable form or shape, in particular an adjustable headrest or a wind turbine blade.

14. A production method for producing a structure (10) according to any one of the preceding claims, comprising of the following steps:
- molding of a first part of the elastically deformable body (12) presenting each internal cavity (14), with each internal cavity (14) being open;
- closing of each internal cavity (14) by means of applying a second part of the elastically deformable body (12) over the first part of the elastically deformable body (12).

15. A method according to claim 14, comprising, at the time of molding, the incorporation of an internal reinforcing framework (50) deployable between a contracted state and a deployed state during the transition of the elastically deformable body (12) passing from the rest configuration to the pressurized configuration, with the internal reinforcing framework (50) in the deployed state locking-in the elastically deformable body (12) in the pressurized configuration.
